(19) Europäisches Patentamt
European Patent Office
Office européen des brevets

(11) Veröffentlichungsnummer: **0 319 847 B1**

(12) **EUROPÄISCHE PATENTSCHRIFT**

(45) Veröffentlichungstag der Patentschrift: **28.07.93**

(51) Int. Cl.[5]: **C07D 213/55**, C07D 405/06, C07D 319/06

(21) Anmeldenummer: **88120057.0**

(22) Anmeldetag: **01.12.88**

(54) **Verfahren zur Herstellung optisch aktiver 3-Desmethylmevalonsäurederivate sowie Zwischenprodukte.**

(30) Priorität: **08.12.87 DE 3741509**

(43) Veröffentlichungstag der Anmeldung: **14.06.89 Patentblatt 89/24**

(45) Bekanntmachung des Hinweises auf die Patenterteilung: **28.07.93 Patentblatt 93/30**

(84) Benannte Vertragsstaaten: **AT BE CH DE ES FR GB GR IT LI LU NL SE**

(56) Entgegenhaltungen:
**WO-A-88/06584**
**US-A- 4 681 893**

**TETRAHEDRON LETTERS, Band 27, Nr. 43, 1986, Seiten 5201-5202, Pergamon Journals Ltd, Oxford, GB; B. O'CONNOR et al.: "Synthesis of argentilactone 11 and goniothalamin 15"**

(73) Patentinhaber: **HOECHST AKTIENGESELLSCHAFT**
**Postfach 80 03 20**
**W-6230 Frankturt am Main 80(DE)**

(72) Erfinder: **Wess, Günther, Dr.**
**Langenselbolder Weg 35**
**W-6455 Erlensee(DE)**
Erfinder: **Kesseler, Kurt, Dr.**
**Gluckstrasse 20A**
**D-6232 Bad Soden am Taunus(DE)**
Erfinder: **Baader, Ekkehard, Dr.**
**Amselweg 14**
**W-6240 Königstein/Taunus(DE)**
Erfinder: **Beck, Gerhard, Dr.**
**Gustav-Freytag-Strasse 24**
**W-6000 Frankturt am Main(DE)**

EP 0 319 847 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Derivate der 3-Desmethylmevalonsäure wie z.B. Mevinolin, sind als Inhibitoren der 3-Hydroxy-3-methylglutaryl-Coenzym-A-Reduktase (HMG-CoA-Reduktase) von großem Interesse. Dieses Enzym katalysiert die Bildung von Mevalonsäure aus 3-Hydroxy-3-methylglutaryl-Coenzym A (HMG-CoA) und spielt als geschwindigkeitsbestimmendes Enzym eine zentrale Rolle bei der Biosynthese des Cholesterins. Derivate der 3-Desmethylmevalonsäure kommen daher zur Senkung hoher Cholesterinspiegel, die mit zahlreichen Erkrankungen in Zusammenhang stehen, in Frage (M. S. Brown, J. L. Goldstein, Science 232, 34 (1986). Neben dem Naturstoff Mevinolin gibt es eine Reihe von strukturell einfacheren Analogen (Drugs of the Future 12, 437 (1987)), die wie Mevinolin die Desmethylmevalonsäure-Teilstruktur enthalten. Diese Verbindungen werden synthetisch gewonnen. Die Desmethylmevalonsäure-Teilstruktur ist ein zentrales Strukturelement dieser Verbindungen und es gibt eine Reihe von Verfahren zu ihrem Aufbau wie die nachfolgend beispielhaft aufgeführten Publikationen zeigen:

D. E. Lynch, R. P. Volante, R. V. Wattley, I. Shinakai, Tetrahedron Lett. 28, 1385 (1987), T. Rosen, C. H. Heathcock, Tetrahedron 42, 4909 (1986), J. D. Prugh, C. S. Rooney, A. A. Deana, H. G. Ramjit, J. Org. Chem. 51, 648 (1986), Y. Guindon, C. Yoakim, M. A. Bernstein, H. E. Morton, Tetrahedron Lett. 26, 1185 (1985), M. Sletzinger, T. R. Verhoeven, R. P. Volante, J. M. McNamara, E. G. Corley, T. M. H. Liu, Tetrahedron Lett. 25, 2951 (1985), J. D. Prugh, A. Deana, Tetrahedron Lett. 23, 281 (1982), Y. L. Yang, J. R. Falck, Tetrahedron Lett. 23, 4305 (1982). Je nach Verfahren fällt die Desmethylmevalonsäure-Teilstruktur in zwei verschiedenen aber synthetisch äquivalenten Formen an:

- als 3,5-Dihydroxycarbonsäurederivat I

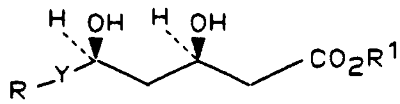

I

oder als $\beta$-Hydroxy-Lakton II

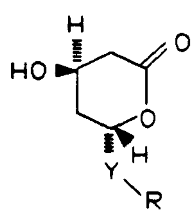

II

Beide Formeln I und II sind nach bekannten Verfahren ineinander umwandelbar. Pharmakologisch sind die Verbindungen interessant, die die in den Formeln I und II angegebenen absoluten Konfigurationen besitzen.

Die Erfindung betrifft ein neues Verfahren zur Herstellung von Verbindungen mit Desmethylmevalonsäure-Teilstruktur in enantiomerenreiner Form der Formeln I und II, worin

Y die $-CH=CH-$ oder $-CH_2-CH_2-$Gruppe ist,

R einen Rest der Formel $\alpha$

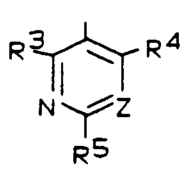

$\alpha$

worin

| | |
|---|---|
| Z | einen Rest der Formel -CH oder ein Stickstoffatom |
| $R^3$, $R^4$, $R^5$ | unabhängig voneinander Wasserstoff, einen geradkettigen oder verzweigten Kohlenwasserstoffrest mit bis zu 6 C-Atomen, der gegebenenfalls am terminalen Kohlenstoff durch |

einen gesättigten oder ungesättigten, cylcischen Kohlenwasserstoffrest mit 3 bis 6 C-Atomen substituiert sein kann, einen cyclischen gesättigten oder bis zu zweifach ungesättigten Kohlenwasserstoffrest mit 3 bis 7 C-Atomen, einen aromatischen Rest ausgewählt aus der Gruppe Phenyl, Furyl, Thienyl, Pyridinyl, der gegebenenfalls im Kern 1 bis 3 gleiche oder verschiedene Substituenten der folgenden Gruppen tragen kann: Halogen, Trifluormethyl, Alkyl oder Alkenyl mit jeweils bis zu 6 C-Atomen, Hydroxy, Alkoxy mit 1 bis 6 C-Atomen, Carboxy, Carbalkoxy mit 1 bis 6 C-Atomen im Alkoxyteil, bedeuten

oder einen Rest der Formel $\beta$

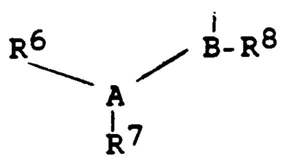

$\beta$

worin

A-B die -CH-CH- oder -C = C-Gruppe

$R^6$ und $R^7$, wobei $R^6$ und $R^7$ gleich oder verschieden sind, einen gesättigten oder ungesättigten Alkylrest mit bis zu 20 C-Atomen, der mit einer Alkoxygruppe mit 1 bis 6 C-Atomen oder der Gruppe

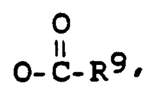

wobei $R^9$ Alkyl mit 1 bis 8 C-Atomen bedeutet, substituiert sein kann, einen Cycloalkylrest mit 3 bis 7 C-Atomen, einen Phenyl-, Thiophen-, Furan-oder Naphthalinrest, wobei die aromatischen Reste im Kern 1- bis 3-fach substituiert sein können mit Halogen, Alkyl oder Alkoxy mit jeweils 1 bis 6 C-Atomen, Cycloalkyl mit 3 bis 7 C-Atomen oder der Gruppe

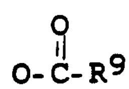

wobei $R^9$ Alkyl mit 1 bis 8 C-Atomen bedeutet, oder einen Pyridinrest, der im Kern 1- bis 3-fach substituiert sein kann mit Alkyl mit 1 bis 4 C-Atomen,

$R^8$ einen gesättigten oder ungesättigten Alkylrest mit bis zu 8 C-Atomen, einen Benzylrest, welcher im Kern 1- bis 3-fach substituiert sein kann mit Halogen, Alkoxy oder Alkyl mit jeweils 1 bis 4 C-Atomen, einen Phenyl-, Thiophen-, Furan-, Naphthalinrest, wobei die aromatischen Reste im Kern 1- bis 3-fach substituiert sein können mit Halogen, Alkoxy oder Alkyl mit jeweils 1 bis 4 C-Atomen, oder einen Cycloalkylrest mit 3 bis 7 C-Atomen, darstellen und

$R^1$ Wasserstoff, ein Metallkation oder Alkyl mit 1 bis 8 C-Atomen ist.

Geeignete Gruppen R werden in den deutschen Offenlegungsschriften DE-A-37 22 807 (Formel $\beta$) und DE-A-38 23 045 (Formel $\alpha$) vorgeschlagen.

Im Vergleich zu beschriebenen Verfahren stellt das erfindungsgemäße Verfahren eine wesentliche Vereinfachung und Verkürzung des Syntheseweges dar.

Ein zentrales Zwischenprodukt für die Durchführung des erfindungsgemäßen Verfahrens ist die Verbindung der Formel III

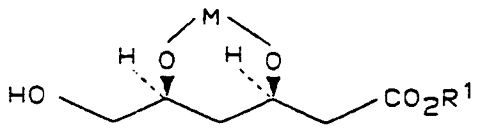

III

worin M eine für 1,3-Diole geeignete Schutzgruppe ist wie z.B. die $H_3C$-C-$CH_3$-Gruppe, oder einen Rest der folgenden Formeln

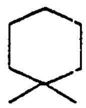

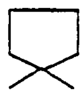

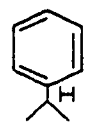

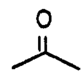

und $R^1$ $C_1$-$C_8$-Alkyl, z.B. t-Butyl, bedeutet, die in verschiedenste Derivate der Formel I bzw. II überführt werden kann.

Das erfindungsgemäße Verfahren ist dadurch gekennzeichnet, daß man

1) einen Diolester der Formel VI

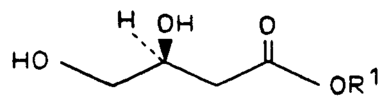

VI

worin $R^1$ $C_1$-$C_8$-Alkyl bedeutet, durch Einführen einer üblichen Schutzgruppe nach an sich bekannten Methoden in eine an der primären Alkoholgruppe geschützte Verbindung der Formel VII

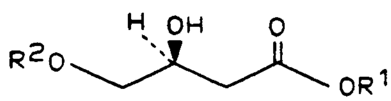

VII

worin $R^1$ $C_1$-$C_8$-Alkyl bedeutet und $R^2$ eine übliche Alkoholschutzgruppe darstellt, überführt

2) eine erhaltene Verbindung der Formel VII oder ihr entsprechendes Alkoholat durch übliche Kondensation mit Essigsäure-t-butylester oder einem geeigneten Äuquivalent wie z.B. Malonester in eine Verbindung der Formel VIII

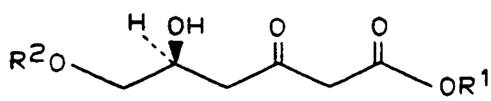

VIII

worin $R^1$ und $R^2$ die zu Formel VII angegebenen Bedeutungen haben, überführt

3) einen erhaltenen Hydroxy-Keto-Ester VIII nach an sich bekannten Methoden zum 1,3-Diol-Ester der Formel IX

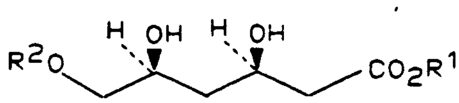

IX

worin $R^1$ und $R^2$ die zu Formel VII angegebenen Bedeutungen haben, reduziert,

4) einen erhaltenen 1,3-Diol-Ester der Formel IX durch Einführen einer für 1,3-Diole geigneten Schutzgruppe in eine Verbindung der Formel X

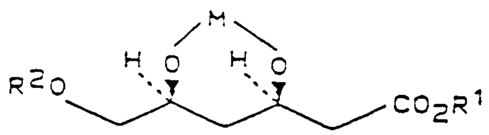

X

worin M eine für 1,3-Diole geeignete Schutzgruppe ist und $R^1$ und $R^2$ die zu Formel VII angegebenen Bedeutungen haben, überführt,

5) eine erhaltene Verbindung der Formel X nach üblichen Methoden unter Abspaltung der Schutzgruppe $R^2$ in eine Verbindung der Formel III

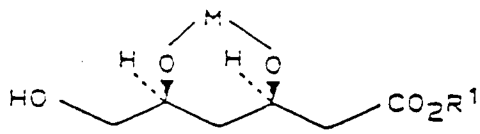

III

worin M eine für 1,3-Diole geeignete Schutzgruppe ist und $R^1$ einen $C_1$-$C_8$-Alkylrest bedeutet, überführt,

6) eine erhaltene Verbindung der Formel III zum Aldehyd der Formel XII

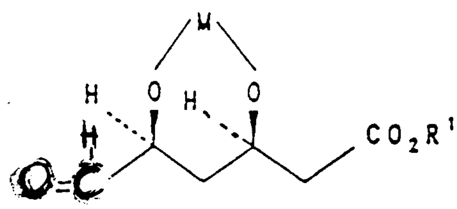

XII

oxydiert

und dann in ein Olefinderivat der Formel XI

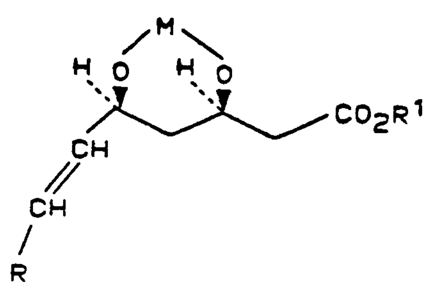

XI

(Y von Formel I = CH = CH)

überführt und

7) eine erhaltene Verbindung der Formel XI hydrolysiert zu einer Verbindung der Formel I, worin Y die CH = CH-Gruppe bedeutet und $R^1$ einen $C_1$-$C_8$-Alkylrest darstellt

8) gegebenenfalls eine erhalten Verbindung der Formel I, worin Y eine CH = CH- Gruppe darstellt, hydriert zu einer Verbindung der Formel I, worin Y die $CH_2$-$CH_2$-Gruppe ist

9) gegebenenfalls eine erhaltene Verbindung in die Säure ($R^1$ = H) oder ein Salz ($R^1$ = Metallkation) überführt und

10) gegebenenfalls eine erhaltene Verbindung der Formel I in eine Verbindung der Formel II überführt.

Falls $R^1$ einen Alkylrest mit 1 bis 8 Kohlenstoffatomen bedeutet, so ist der Alkylrest geradkettig oder verzweigt.

Die Ausgangsverbindungen der Formel VI sind bekannt oder können nach üblichen Methoden hergestellt werden. Bei der Herstellung geht man zweckmäßigerweise aus von L(-)-Äpfelsäure [S(-)-2-Hydroxybernsteinsäure] der Formel IV

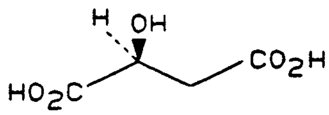

IV

Diese wird nach bekannten Methoden in einen $C_1$-$C_8$-Alkylester der Formel V

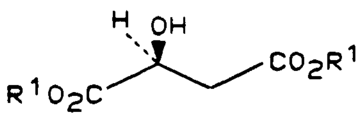

V

worin $R^1$ $C_1$-$C_8$-Alkyl, vorzugsweise Methyl oder Ethyl, bedeutet, überführt, vorzugsweise durch Behandeln mit Alkohol unter sauren Bedingungen.

Verbindung V wird zum Diol-Ester VI reduziert, vorzugsweise mit Boran-Dimethylsulfid-Komplex unter Verwendung einer katalytischen Menge Natriumborhydrid nach einem Verfahren von T. Moriwake et al. (Chemistry Letters 1984, 1389).

Bei dem erfindungsgemäßen Verfahren wird der Diol-Ester VI im ersten Schritt an der primären Alkoholgruppe geschützt. In der Formel VII bedeutet $R^2$ eine Alkoholschutzgruppe (vgl. Protective Groups in Organic Synthesis, Wiley, New York 1981), vorzugsweise t-Butyldiphenylsilyl.

Die Kondensation gemäß Schritt 2 wird z.B. mit Essigsäure-t-butylester, vorzugsweise mit dem Lithiumenolat des Essigsäure-t-butylesters, in einem Lösungsmittel bei -78 °C bis Raumtemperatur, vorzugsweise zwischen -30 °C und -10 °C, in Tetrahydrofuran (THF) durchgeführt. Es werden 2 bis 5 Äquivalente des Enolates verwendet. Das Lithiumenolat wird nach den üblichen Methoden bereitet, vorzugsweise mit Lithiumdiisopropylamid (LDA) bei -70 °C bis -50 °C. Eine Variante des Verfahrens kann darin bestehen, daß die Verbindung VII in Form ihres Alkoholates eingesetzt wird, und zwar z.B. als Lithium-, Natrium- oder Magnesiumalkoholat.

Hydroxy-Keto-Ester VIII wird zum 1,3-Diol-Ester IX reduziert und zwar so, daß bevorzugt oder ausschließlich die in Formel IX angegebene Konfiguration entsteht.

Dazu verwendet man vorzugsweise ein Alkylboran oder Alkoxyalkylboran in Verbindung mit Natriumborhydrid bei Temperaturen zwischen -110 °C und 0 °C, und zwar in Anlehnung an bekannte Literaturverfahren (K. Narasaka, H. C. Pai, Chem. Lett. 1980, 1415, Tetrahedron Lett. 28, 155 (1987)).

Verbindung IX wird zweckmäßigerweise in das Acetonid X

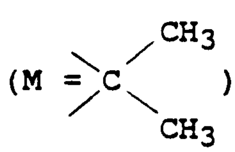

überführt, vorzugsweise unter Verwendung von 2,2-Dimethoxypropan in Aceton unter Zusatz eines sauren Katalysators, wie z.B. p-Toluolsulfonsäure.

Das Acetonid X wird in den Alkohol III

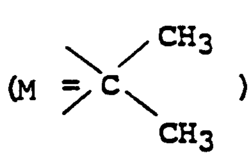

überführt. Zur Abspaltung der Schutzgruppen $R^2$ werden an sich bekannten Verfahren angewendet. t-Butyldiphenylsilyl wird vorzugsweise mit Fluoridionen absgespalten, z.B. mit Tetrabutylammoniumfluorid in THF.

Der Alkohol III ist ein wertvoller Synthesebaustein für die Herstellung von Desmethylmevalonsäurederivaten.

Zur Herstellung der Olefinderivate der Formel XI wird III zum Aldehyd XII oxidiert, z.B. mit Dimethylsulfoxid/Oxalylchlorid/Triethylamin (Synthesis 1981, 165)

XII

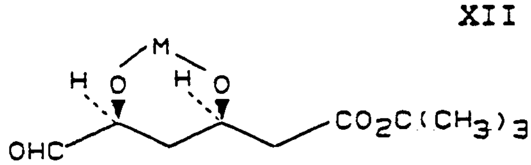

$(R^1 = C(CH_3)_3)$

Die Verknüpfung von XII, worin M z.B.

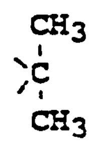

ist, mit einer geeigneten Verbindung, z. B. der Formel XIV oder XV z. B. zur Herstellung von Verbindungen entsprechend der deutschen Offenlegungsschrift DE-A-38 23 045

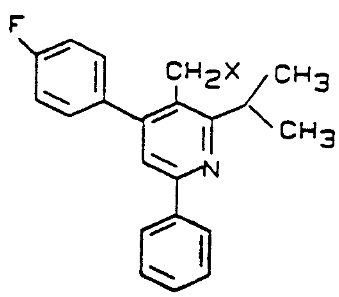

XIV X = $PPh_3{}^+Br^\ominus$

XV X = $PO(OAk)_2$

Ak = $C_1$-$C_4$-Alkyl

erfolgt vorzugsweise durch eine Wittig- bzw. Wittig-Horner-Reaktion über die entsprechenden Phosphoniumhalogenide XIV oder Alkylphosphonate XV zu Verbindung XI′ der Formel

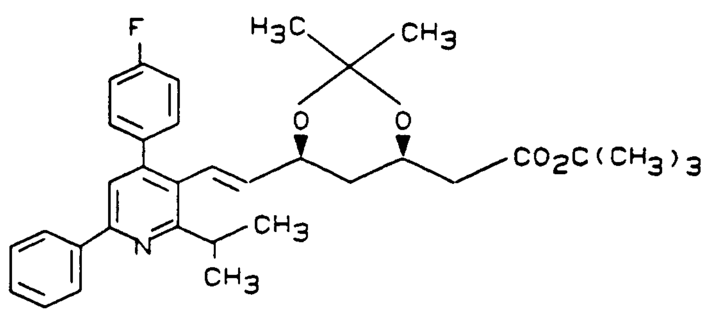

XI'

In analoger Weise können weitere Verbindungen XI hergestellt werden. Verbindungen XI werden nach an sich bekannten Methoden zu Verbindungen I mit Y = CH = CH hydrolysiert. Die Überführung einer Verbindung XI in ein gewünschtes Endprodukt wird nachfolgend an der Reaktion mit Verbindung XI′ erläutert. Hydrolyse des Acetonids der Formel XI′ unter sauren Bedingungen führt zu Verbindung I′

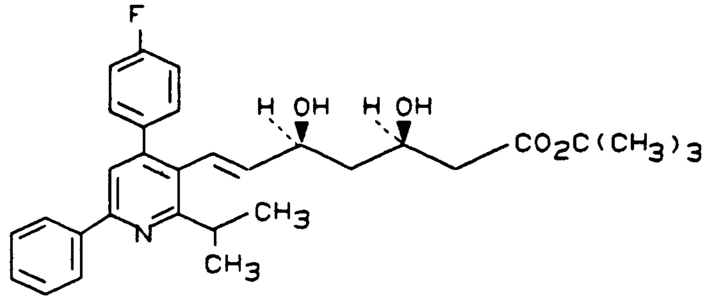

I'

I' wird nach Standardverfahren in das entsprechende Lakton

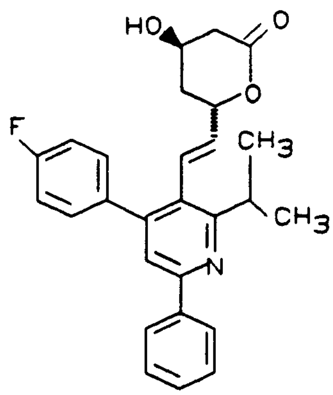

II'

überführt, vorzugsweise mit Trifluoressigsäure in Methylenchlorid.

Die Überführung von Verbindungen in Laktone II erfolgt entweder direkt aus den Estern ($R^1$ = $C_1$-$C_8$-Alkyl) oder aus der entsprechenden freien Säure ($R^1$ = H). Weiterhin werden Laktone säureempfindlicher Verbindungen aus den freien Säuren durch Verwendung von Laktonisierungsreagenzien wie z.B. Carbodi imiden hergestellt.

Die Aldehyde der Formel XII, worin M einen Rest der Formel

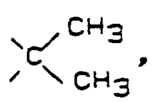, 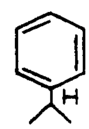 oder 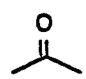

bedeutet, sind neu.

Die Erfindung betrifft daher auch diese Verbindungen sowie Verfahren zu ihrer Herstellung. Die Aldehyde sind z.B. wertvolle Zwischenprodukte zur Herstellung von Verbindungen der Formel I und der Formel II, die wertvolle Arzneimittel darstellen.

**Beispiel 1**

**S-3-Hydroxy-4-(t-butyldiphenylsilyloxy)buttersäuremethylester (Formel VII)**

Zu einer Lösung von 31.1 g (0.233 mol) (3S)-3,4-Dihydroxybuttersäuremethylester(Formel VI), Chem. Lett. 1984, 1389, und 31.7 g (0.466 mol) Imidazol in 400 ml trockenem Dimethylformamid tropfte man bei 0 °C 64.05 g (0.233 mol) t-Butyldiphenylchlorsilan. Es wurde 2 h bei Raumtemp. gerührt, anschließend mit 1000 ml Wasser versetzt und mit Ether extrahiert ( 2 x). Die vereinigten organischen Phasen wurden getrocknet ($MgSO_4$) und einrotiert. Flash-Chromatographie auf Kieselgel (Cyclohexan/Essigester = 3 : 1 + 1 % $NEt_3$) ergab 77.4 g (0.208 mol, 89 %) Beispiel 1.

$[\alpha]_D^{20}$ = - 9.6 ° (c=10,2 in Methanol)
H-NMR ($CDCl_3$), 60 MHz): δ = 7,80 - 7,20 (m, 10H), 4,20 - 3,60 (m, 6H), 2,5 (d, 2H), 1.05 (s, 9H)

**Beispiel 2**

**(5S)-5-Hydroxy-3-oxo-6-(t-butyldiphenylsilyloxy)hexansäure-t-butylester (Formel VIII)**

Zu einer Lösung von 41.0 g (0.405 mol) Diisopropylamin in 400 ml trockenem THF wurden bei 0°C unter Argon 225 ml (0.36 mol) n-Butyllithium (Hexan) getropft. Nach 30 min bei 0°C wurde auf - 70°C gekühlt und 36,7 ml (0.27 mol) Essigsäure-t-butylester getropft.

Nach 1 h bei - 70°C wurden 27,9 g (0.075 mol) der Verbindung gemäß Beispiel 1 in wenig THF gelöst zugetropft. Nach 1.5 h bei - 70°C ließ man langsam auf - 15°C kommen. Schließlich rührte man nach 15 min bei - 15°C und goß dann auf kalte 500 ml 2 n HCl/500 ml Ether. Die wäßrige Phase wurde 2 x mit Ether extrahiert und die vereinigten organischen Phasen mit gesättigter Natriumchloridlösung neutral gewaschen (3 x). Trocknen mit $MgSO_4$ und Abziehen des Lösungsmittels im Vakuum ergab 36 g (quantitativ) der Titelverbindung. Sie wurde ohne weitere Reinigung zur nächsten Stufe umgesetzt.
$[\alpha]_D^{20}$ = - 9,8° (c = 10,6 in Methanol) für das Rohprodukt.

**Beispiel 3**

**3R, 5S-Dihydroxy-6-(t-butyldiphenylsilyloxy)hexansäure-t-butylester (Formel IX)**

Zu 9,13 g (0.02 mol) der Verbindung aus Beispiel 2 in 200 ml trockenem THF wurden unter Argon 32 ml (0.032 mol) Triethylboranlösung (THF) bei Raumtemperatur getropft. Nach 15 min Rühren bei Raumtemp. wurde auf -70°C gekühlt und 1.51 g (0.04 mol) Natriumborhydrid zugegeben und anschließend 15 ml trockenes Methanol zugegeben. Man ließ 2.5 h bei - 70°C rühren und goß anschließend auf eine kalte Lösung von 35 ml 35 proz. Wasserstoffperorid in 300 ml Wasser. Extraktion mit Essigester (3 x). Die vereinigten organischen Phasen wurden mit gesättigter Natriumhydrogencarbonatlösung gewaschen (3 x) und getrocknet ($MgSO_4$). Abziehen des Lösungsmittels im Vakuum ergab 9.48 g (quant.) der Titelverbindung.

Eine analytische Probe wurde durch Flash-Chromatographie auf Kieselgel (Essigester/Cyclohexan = 2 : 1 + 1 % $NEt_3$) gereinigt.
$[\alpha]_D^{20}$ = - 6.6 ° (c = 10.4 in Methanol)
H-NMR ($CDCl_3$, 270 MHz): δ = 7,15 und 7,40 (jeweils m, zusammen 10H), 4,21 (m, 1H), 4.0 (m, 1H), 3.51 (m, 2H), 2.40 (m, 2H), 1.70 - 1.40 und 1.05 (mehrere m, zusammen 20H)

**Beispiel 4**

**(3R, 5S)-6-(t-Butyldiphenylsilyloxy)-3,5-O-isopropyliden-3,5-dihydroxyhexansäure-t-butylester (Formel X)**

6.88 g (0.015 mol) der Verbindung aus Beispiel 3 (Rohprodukt) wurden in 200 ml Aceton/2,75 ml 2,2-Dimethoxypropan gelöst und bei Raumtemp. mit 250 mg p-Toluolsulfonsäure versetzt. Nach 2 h bei Raumtemp. wurden 4 ml Triethylamin zugegeben und das Lösungsmittel im Vakuum entfernt. Der Rückstand wurde zwischen Ether und Wasser verteilt. Die wäßrige Phase wurde einmal mit Ether extrahiert. Die vereinigten organischen Phasen wurden mit Natriumhydrogencarbonatlösung gewaschen und getrocknet ($MgSo_4$). Abziehen des Lösungsmittels im Vakuum und Flash-Chromatographie auf Kieselgel (Cyclohexan/Essigester = 5 : 1) ergab 5.2 g (0.010 mol, 70 % der Titelverbindung.
$[\alpha]_D^{20}$ = -4.0 (c = 24.6 in Methanol)
H-NMR (270 MHz, $CDCl_3$): δ = 7,20 und 7,40 (jeweils m, zusammen 10H), 4.25 (m, 1H), 4.00 (m, 1H), 3.70 (dd, 1H), 3.52 (dd, 1H), 2.45 (dd, 1H), 2.30 (dd, 1H), 1.70 (dt, 1H), 1.50 - 1.00 (verschiedene m, zusammen 25 H)

**Beispiel 5**

**(3R, 5S)-6-Hydroxy-3,5-O-isopropyliden-3,5-dihydroxyhexansäure-t-butylester (Formel III)**

Zu einer Lösung von 2,49 g (5 mmol) der Verbindung aus Beispiel 4 in 20 ml THF wurden bei 0 °C 1,89 g (6 mmol) Tetrabutylammoniumfluorid-Trihydrat zugegeben. Nach 3 h bei 0 °C wurde mit 100 ml Ether verdünnt und die Lösung mit 100 ml gesättigter Natriumchloridlösung gewaschen. Die wäßrige Phase wurde einmal mit Ether reextrahiert und die vereinigten organischen Phasen getrocknet ($MgSO_4$). Das Lösungsmittel wurde im Vakuum entfernt und der Rückstand auf Kieselgel Flash-chromatographiert (Cyclohexan/Essigester = 1 : 1). Ausbeute 1.04 g (4,0 mmol, 80 %).
$[\alpha]_D^{20}$ = -3.7 (c = 14.9 in Methanol)
H-NMR (DMSO-$d_6$, 270 MHz): $\delta$ = 4,61 (t, 1H), 4,20 (m, 1H), 3,88 (m, 1H), 3,40 - 3,20 (m, 2H), 2,38 (dd, 1H), 2,22 (dd, 1H), 1,55 (dt, 1H), 1,40 (s, 12H), 1,25 (s, 3H), 1,15 (m, 1H)
MS: $C_{13}H_{24}O_5$, 261 (M + $H^+$)

**Beispiel 6**

**(3R, 5S)-6-Oxo-3,5-O-isopropyliden-3,5-dihydroxyhexansäure-t-butylester (Formel XII)**

Eine Lösung von 0.235 ml (2.75 mmol) Oxalylchlorid in 10 ml Dichlormethan wurde bei - 78 °C tropfenweise mit 0.43 g (5.50 mmol) Dimethylsulfoxid versetzt und 5 min bei gleicher Temperatur gerührt. Anschließend wurden 0.65 g (2.5 mmol) der Verbindung gemäß Beispiel 5 zugetropft. Nach 5minütigem Rühren wurden 1.70 ml Triethylamin zugegeben, und das Reaktionsgemisch wurde in 2 h auf Raumtemperatur gebracht. Zur Aufarbeitung wurde der Ansatz auf Wasser gegeben und 3 x mit je 50 ml Ether ausgeschüttelt. Die vereinigten organischen Extrakte wurden über $MgSo_4$ getrocknet und eingedampft. Das zurückbleibende Öl wurde im Feinvakuum von flüchtigen Bestandteilen befreit und sofort weiter umgesetzt.
Rf (Cyclohexan/Essigester = 1 : 1) : 0,24

**Beispiel 7**

**E-6S-2-(4-(4-Fluorphenyl)-2-(1-methylethyl)-6-phenylpyridin-3-yl)-ethenyl-4R-hydroxy-3,4,5,6-tetrahydro-2H-pyran-2-on (Formel II)**

Schritt a: (Formel XI)

Eine Lösung von 0.26 g (2.5 mmol) Diisopropylamin in 10 ml THF wurde bei 0 °C tropfenweise mit 1.6 ml (2.5 mmol) einer 1.6 M Lösung von n-Butyl-lithium in Hexan versetzt und 15 min bei gleicher Temperatur gerührt. Zu dieser Lösung wurden 1.10 g (2.5 mmol) Diethyl- (4- (4-fluorphenyl)-2-(1-methylethyl)-6-phenylpyridin -3- yl-methyl)phosphonat (Formel XV, hergestellt durch 8 h Erhitzen des entsprechenden Bromids mit Triethylphosphit in Toluol und chromatographischer Reinigung (Cyclohexan/ Ethylacetat = 2 : 1, Silicagel) ) in 5 ml THF getropft. Die resultierende tiefgrüne Reaktionslösung wurde 1 h bei 0 °C gerührt, anschließend mit 0.65 g des Rohproduktes von Beispiel 6 versetzt und unter Rühren in 3 h auf Raumtemperatur gebracht. Zur Aufarbeitung wurde das Gemisch auf 100 ml Wasser gegeben und 3 x mit je 100 ml Ether extrahiert. Die vereinigten organischen Extrakte wurden mit ges. Kochsalzlösung gewaschen, getrocknet ($MgSo_4$) und eingedampft. Flash-Chromatographie auf Kieselgel (Cyclohexan/Essigester = 3 : 1 + 1 % $NEt_3$).
H-NMR (DMSO-$d_6$; 270 MHz): $\delta$ = 0.93(mc, 2H), 1.25 (s, 3H), 1.31 (d, J = 7Hz, 6H), 1.41 (s, 9H), 1.43 (s, 3H), 2.48 (mc, 2H), 3.52 (h, J = 7Hz, 1H), 4.30 (mc, 1H), 4.47 (mc, 1H), 5.33 (dd, J = 16Hz, 6Hz, 1H), 6.56 (d, J = 16Hz, 1H), 7.25 (mc, 2H), 7.40 - 7.53 (m, 5H), 7.66 (s, 1H), 8.16 (mc, 2H) ppm
MS (DCI): m/e = 546 ($M^{\oplus}$ + H)

**Schritt b:**

**E, 3R, 5S-3,5-Dihydroxy-7-(4-(4-fluorphenyl)-2-(1-methylethyl)-6-phenylpyridin-3-yl)hept-6-ensäure-t-butylester (Formel I)**

107 mg (0.19 mmol) Verbindung aus Schritt 7a wurden in 10 ml THF gelöst und mit 5 ml 2 n HCl versetzt. Nach 1.5 h bei Raumtemp. wurde mit gesättigter Natriumhydrogencarbonatlösung neutralisiert und

mit Ether extrahiert. Die vereinigten Etherphasen wurden einmal mit gesättigter Natriumchloridlösung gewaschen und getrocknet ($MgSO_4$). Abziehen des Lösungsmittels und Kristallisation aus Diisopropylether/n-Pentan ergab 78 mg (0.15 mmol, 79 %).

$^1$H-NMR ($CDCl_3$; 270 MHz): δ = 1.36 (d, J = 7 Hz, 6H), 1.43 - 1.55 (m, 11H), 2.35 (s, 1H), 2.37 (d, J = 2Hz, 1H), 3.30 (brs, 1H), 3.46 (h, J = 7Hz, 1H), 3.73 (brs, 1H), 4.11 (mc, 1H), 4.41 (mc, 1H), 5.38 (dd, J = 16Hz, 7Hz, 1H), 6.62 (dd, J = 16Hz, 2 Hz, 1H), 7.08 (mc, 2H), 7.25 - 7.49 (m, 6H), 8.10 (mc, 2H)

MS: (FAB): m/e = 506 ($M^{\oplus}$ + H)

**Schritt c:**

**E-6S-2-(4-(4-Fluorphenyl)-2-(1-methylethyl)-6-phenylpyridin-3-yl)ethenyl-4R-hydroxy-3, 4, 5, 6-tetrahydro-2H-pyran-2-on (Formel II)**

Eine Lösung von 41 mg (0.08 mmol) Verbindung aus Schritt b in 2 ml Dichlormethan und 0.10 ml (0.59 mmol) Trifluoressigsäure wurde bei Raumtemperatur gerührt, wobei das Fortschreiten der Umsetzung dünnschichtchromatographisch kontrolliert wurde (Silicagel, Cyclohexan/Ethylacetat = 2 : 1). Nach 8 h war kein Edukt mehr vorhanden. Die Reaktionslösung wurde auf ges. $NaHCO_3$- Lösung gegeben und mehrfach ausgeethert. Die vereinig- ten organischen Extrakte wurden mit Wasser gewaschen, getrocknet ($MgSO_4$) und eingedampft. Zurück blieben 40 mg (100 %) Beispiel 7, das in allen Merkmalen mit authentischem Material übereinstimmt (vergl. deutsche Offenlegungsschrift DE-A-38 23 045).

**Schritt d:**

Aus den Estern gemäß Schritt b erhält man die entsprechenden Natriumsalze:

**E, 3R, 5S-3,5-Dihydroxy-7-(4-(4-fluorphenyl)-2-(1-methylethyl)-6-phenylpyridin-3-yl)hept-6-ensäure Natriumsalz**

1 mmol Ester gemäß Schritt b wird mit 1 mmol Natronlauge in Ethanol/Wasser bei Raumtemp. verseift. Das Lösungsmittel wird im Vakuum entfernt und der Rückstand mehrmals mit Toluol abgeraucht. Der Rückstand wird mit Ether/Hexan verrieben.

In analoger Weise können die folgenden Verbindungen der Formel I bzw. II hergestellt werden:

E, 3R, 5S-9,9-Di-(4-fluorphenyl)-3,5-dihydroxy-8-isopropyl-6,8-nonadiensäure Natriumsalz
E, 3R, 5S-9,9-Di-(4-fluor-3-methylphenyl)-3,5-dihydroxy-8-isopropyl-6,8-nonadiensäure Natriumsalz
E, 3R, 5S-9,9-Di-(4-fluor-2-methylphenyl)-3,5-dihydroxy-8-isopropyl-6,8-nonadiensäure Natriumsalz
E, 3R, 5R-9,9-Di-(4-fluor-3-methylphenyl)-3,5-dihydroxy-8-isopropyl-8-nonensäure Natriumsalz
E, 3R, 5R-9,9-Di-(4-fluorphenyl)-3,5-dihydroxy-8-isopropyl-8-nonensäure Natriumsalz
E-6S-(2-(4-(4-Fluorphenyl)-2-(1-methylethyl)-6-phenylpyridin-3-yl)ethenyl)-4R-hydroxy-3,4,5,6-tetrahydro-2H-pyran-2-on
E-6S-(2-(2,6-Bis-(1-methylethyl)-4-(4-fluorphenyl)-pyridin-3-yl)ethenyl)-4R-hydroxy-3,4,5,6-tetrahydro-2H-pyran-2-on
E-6S-(2-(6-(1,1 Dimethylethyl)-4-(4-fluorphenyl)-2-(1-methylethyl)pyridin-3-yl)ethenyl)-4R-hydroxy-3,4,5,6-tetrahydro-2H-pyran-2-on
E-6S-(2-(4,6-Bis-(4-fluorphenyl)-2-(1-methylethyl)pyridin-3-yl)ethenyl)-4R-hydroxy-3,4,5,6-tetrahydro-2H-pyran-2-on
E-6S-(2-(2-(4-Fluorphenyl)-4-(1-methylethyl)-6-phenylpyridin-3-yl)ethenyl)-4R-hydroxy-3,4,5,6-tetrahydro-2H-pyran-2-on
E-6S-(2-(6-(2,5-Dimethylphenyl)-4-(4-fluorphenyl)-2-(1-methylethyl)pyridin-3-yl)ethenyl)-4R-hydroxy-3,4,5,6-tetrahydro-2H-pyran-2-on
6S-(2-(2-Ethyl-4-(4-fluorphenyl)-6-phenylpyridin-3-yl) ethyl)-4R-hydroxy-3,4,5,6-tetrahydro-2H-pyran-2-on
E-6S-(2-(2-Cyclohexyl-4-(4-fluorphenyl)-6-(1-methylethyl)pyridin-3-yl)ethenyl)-4R-hydroxy-3,4,5,6-tetrahydro-2H-pyran-2-on
E-6S-(2-(4-(4-Fluorphenyl)-2-(1-methylethyl)-6-phenylpyrimidin-3-yl)ethenyl)-4R-hydroxy-3,4,5,6-tetrahydro-2H-pyran-2-on)
E-6S-(2-(2,6-Bis-(1-methylethyl)-4-(4-fluorphenyl)pyrimidin-3-yl)ethenyl)-4R-hydroxy-3,4,5,6-tetrahydro-2H-pyran-2-on
E-6S-(2-(6-Cyclohexyl-4-(4-fluorphenyl)-2-(1-methylethyl)pyrimidin-3-yl)ethenyl)-4R-hydroxy-3,4,5,6-tetrahydro-2H-pyran-2-on

E-6S-(2-(4,6-Bis-(4-fluorphenyl)-2-(1-methylethyl)pyrimidin-3-yl)ethenyl)-4R-hydroxy-3,4,5,6-tetrahydro-2H-pyran-2-on.

**Patentansprüche**

1. Verfahren zur Herstellung von optisch aktiven 3-Desmethylmevalonsäurederivaten der Formel I

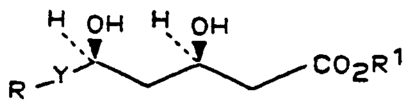

I

(3,5-Dihydroxycarbonsäurederivate) oder der Formel II

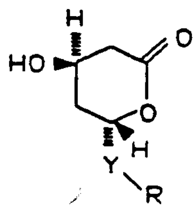

II

(β-Hydroxy-Lactone)
worin
Y die -CH=CH- oder $-CH_2-CH_2-$Gruppe ist,
R einen Rest der Formel α

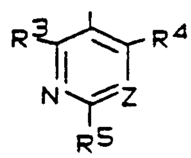

α

worin

| | |
|---|---|
| Z | einen Rest der Formel -CH oder ein Stickstoffatom |
| $R^3$, $R^4$, $R^5$ | unabhängig voneinander Wasserstoff, einen geradkettigen oder verzweigten Kohlenwasserstoffrest mit bis zu 6 C-Atomen, der gegebenenfalls am terminalen Kohlenstoff durch einen gesättigten oder ungesättigten, cylcischen Kohlenwasserstoffrest mit 3 bis 6 C-Atomen substituiert sein kann, einen cyclischen gesättigten oder bis zu zweifach ungesättigten Kohlenwasserstoffrest mit 3 bis 7 C-Atomen, einen aromatischen Rest ausgewählt aus der Gruppe Phenyl, Furyl, Thienyl, Pyridinyl, der gegebenenfalls im Kern 1 bis 3 gleiche oder verschiedene Substituenten der folgenden Gruppen tragen kann: Halogen, Trifluormethyl, Alkyl oder Alkenyl mit jeweils bis zu 6 C-Atomen, Hydroxy, Alkoxy mit 1 bis 6 C-Atomen, Carboxy, Carbalkoxy mit 1 bis 6 C-Atomen im Alkoxyteil, bedeuten |

oder einen Rest der Formel β

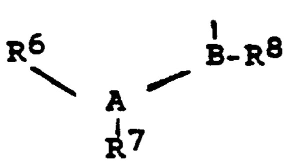

β

worin

A-B die -CH-CH- oder -C = C-Gruppe

$R^6$ und $R^7$, wobei $R^6$ und $R^7$ gleich oder verschieden sind, einen gesättigten oder ungesättigten Alkylrest mit bis zu 20 C-Atomen, der mit einer Alkoxygruppe mit 1 bis 6 C-Atomen oder der Gruppe

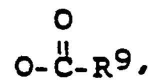

wobei $R^9$ Alkyl mit 1 bis 8 C-Atomen bedeutet, substituiert sein kann, einen Cycloalkylrest mit 3 bis 7 C-Atomen, einen Phenyl-, Thiophen-, Furan- oder Naphthalinrest, wobei die aromatischen Reste im Kern 1- bis 3-fach substituiert sein können mit Halogen, Alkyl oder Alkoxy mit jeweils 1 bis 6 C-Atomen, Cycloalkyl mit 3 bis 7 C-Atomen oder der Gruppe

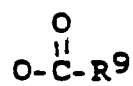

wobei $R^9$ Alkyl mit 1 bis 8 C-Atomen bedeutet, oder einen Pyridinrest, der im Kern 1- bis 3-fach substituiert sein kann mit Alkyl mit 1 bis 4 C-Atomen,

$R^8$ einen gesättigten oder ungesättigten Alkylrest mit bis zu 8 C-Atomen, einen Benzylrest, welcher im Kern 1- bis 3-fach substituiert sein kann mit Halogen, Alkoxy oder Alkyl mit jeweils 1 bis 4 C-Atomen, einen Phenyl-, Thiophen-, Furan-, Naphthalinrest, wobei die aromatischen Reste im Kern 1- bis 3-fach substituiert sein können mit Halogen, Alkoxy oder Alkyl mit jeweils 1 bis 4 C-Atomen, oder einen Cycloalkylrest mit 3 bis 7 C-Atomen, darstellen und

$R^1$ Wasserstoff, ein Metallkation oder Alkyl mit 1 bis 8 C-Atomen ist, dadurch gekennzeichnet, daß man

1) einen Diolester der Formel VI

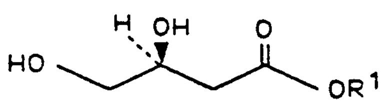

VI

worin $R^1$ $C_1$-$C_8$-Alkyl bedeutet, durch Einführen einer üblichen Schutzgruppe nach an sich bekannten Methoden in eine an der primären Alkoholgruppe geschützte Verbindung der Formel VII

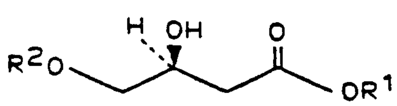

VII

worin $R^1$ $C_1$-$C_8$-Alkyl bedeutet und $R^2$ eine übliche Alkoholschutzgruppe darstellt, überführt

2) eine erhaltene Verbindung der Formel VII oder ihr entsprechendes Alkoholat durch übliche Kondensation mit Essigsäure-t-butylester oder einem geeigneten Äuqivalent in eine Verbindung der Formel VIII

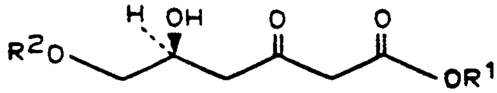

VIII

worin $R^1$ und $R^2$ die zu Formel VII angegebenen Bedeutungen haben, überführt
3) einen erhaltenen Hydroxy-Keto-Ester VIII nach an sich bekannten Methoden zum 1,3-Diol-Ester der Formel IX

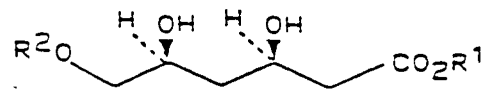

IX

worin $R^1$ und $R^2$ die zu Formel VII angegebenen Bedeutungen haben, reduziert,
4) einen erhaltenen 1,3-Diol-Ester der Formel IX durch Einführen einer für 1,3-Diole geeigneten Schutzgruppe in eine Verbindung der Formel X

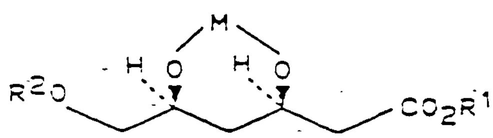

X

worin M eine für 1,3-Diole geeignete Schutzgruppe ist und $R^1$ und $R^2$ die zu Formel VII angegebenen Bedeutungen haben, überführt,
5) eine erhaltene Verbindung der Formel X nach üblichen Methoden unter Abspaltung der Schutzgruppe $R^2$ in eine Verbindung der Formel III

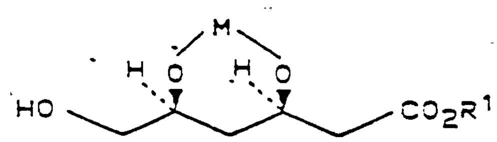

III

worin M eine für 1,3-Diole geeignete Schutzgruppe ist und $R^1$ einen $C_1$-$C_8$-Alkylrest bedeutet, überführt,
6) eine erhaltene Verbindung der Formel III zum Aldehyd der Formel XII

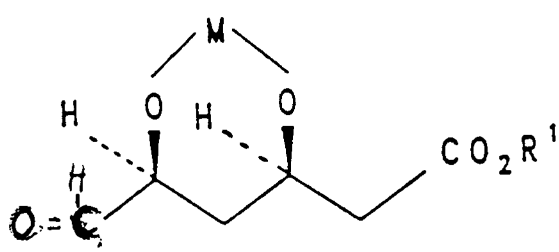

XII

oxydiert und dann in ein Olefinderivat der Formel XI

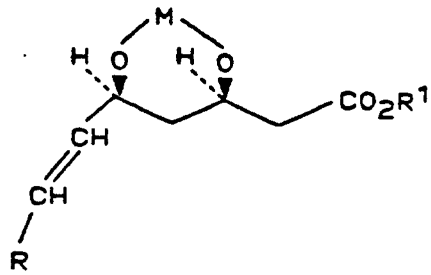

XI

(Y von Formel I = CH = CH)

überführt und

7) eine erhaltene Verbindung der Formel XI hydrolysiert zu einer Verbindung der Formel I, worin Y die CH = CH-Gruppe bedeutet und $R^1$ einen $C_1$-$C_8$-Alkylrest darstellt

8) gegebenenfalls eine erhalten Verbindung der Formel I, worin Y eine CH = CH-Gruppe darstellt, hydriert zu einer Verbindung der Formel I, worin Y die $CH_2$-$CH_2$-Gruppe ist

9) gegebenenfalls eine erhaltene Verbindung in die Säure ($R^1$ = H) oder ein Salz ($R^1$ = Metallkation) überführt und

10) gegebenenfalls eine erhaltene Verbindung der Formel I in eine Verbindung der Formel II überführt.

**2.** Aldehyde der Formel XII

XII

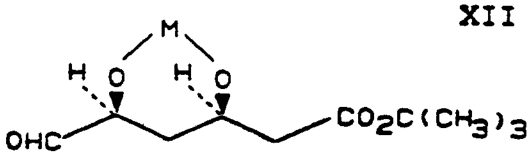

worin M einen Rest der Formel

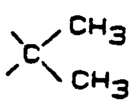 , 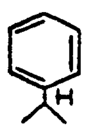 oder 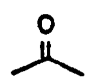 bedeutet.

**3.** Verfahren zur Herstellung von Aldehyden der Formel XII, dadurch gekennzeichnet, daß man Alkohole der Formel III, worin M die zu Formel XII angegebenen Bedeutungen hat, oxydiert.

**Claims**

**1.** A process for the preparation of optically active 3-demethylmevalonic acid derivatives of the formula I

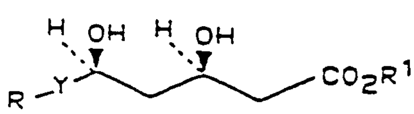

I

(3,5-dihydroxy carboxylic acid derivatives) or of the formula II

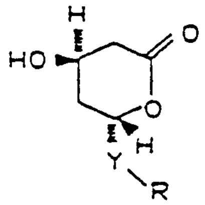

II

(β-hydroxy lactones)
in which
Y is the -CH=CH- or $-CH_2-CH_2-$ group,
R is a radical of the formula α

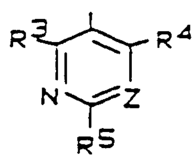

α

in which

Z denotes a radical of the formula -CE or a nitrogen atom,

$R^3$, $R^4$ and $R^5$ denote, independently of one another, hydrogen, a straight-chain or branched hydrocarbon radical which has up to 6 carbon atoms and can optionally be substituted at the terminal carbon by a saturated or unsaturated cyclic hydrocarbon radical having 3 to 6 carbon atoms, or denote a cyclic saturated or up to doubly unsaturated hydrocarbon radical having 3 to 7 carbon atoms, an aromatic radical selected from the group comprising phenyl, furyl, thienyl and pyridinyl, which can optionally carry in the nucleus 1 to 3 identical or different substituents from the following groups: halogen, trifluoromethyl, alkyl or alkenyl, having up to 6 carbon atoms in each case, hydroxyl, alkoxy having 1 to 6 carbon atoms, carboxyl, or carbalkoxy having 1 to 6 carbon atoms in the alkoxy moiety,

or is a radical of the formula β

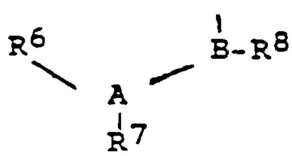

β

in which

A-B represents the -CH-CH- or -C=C- group

$R^6$ and $R^7$, with $R^6$ and $R^7$ being identical or different, represent a saturated or unsaturated alkyl radical which has up to 20 carbon atoms and can be substituted by an alkoxy group having 1 to 6 carbon atoms or the group

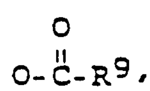

where $R^9$ denotes alkyl having 1 to 8 carbon atoms, or represents a cycloalkyl radical having 3 to 7 carbon atoms, a phenyl, thienyl, furyl or naphthyl radical, it being possible for the aromatic radicals to be substituted in the nucleus 1 to 3 times by

halogen, alkyl or alkoxy, having 1 to 6 carbon atoms in each case, cycloalkyl having 3 to 7 carbon atoms or the group

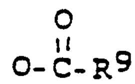

where $R^9$ denotes alkyl having 1 to 8 carbon atoms, or a pyridinyl radical which can be substituted in the nucleus 1 to 3 times by alkyl having 1 to 4 carbon atoms,

$R^8$ represents a saturated or unsaturated alkyl radical having up to 8 carbon atoms, a benzyl radical which can be substituted in the nucleus 1 to 3 times by halogen, alkoxy or alkyl, having 1 to 4 carbon atoms in each case, a phenyl, thienyl, furyl or naphthyl radical, it being possible for the aromatic radicals to be substituted in the nucleus 1 to 3 times by halogen, alkoxy or alkyl, having 1 to 4 carbon atoms in each case, or a cycloalkyl radical having 3 to 7 carbon atoms, and

$R^1$ is hydrogen, a metal cation or alkyl having 1 to 8 carbon atoms, which comprises

1) converting a diol ester of the formula VI

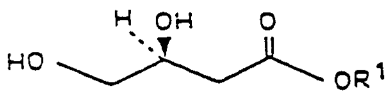

VI

in which $R^1$ denotes $C_1$-$C_8$-alkyl, by introduction of a customary protective group by methods known per se into a compound of the formula VII which is protected on the primary alcohol group

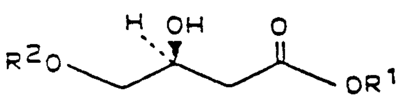

VII

and in which $R^1$ denotes $C_1$-$C_8$-alkyl and $R^2$ represents a customary alcohol protective group,

2) converting a resulting compound of the formula VII, or the corresponding alcoholate thereof, by customary condensation with t-butyl acetate, or a suitable equivalent into a compound of the formula VIII

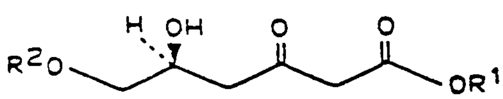

VIII

in which $R^1$ and $R^2$ have the meanings indicated for formula VII,

3) reducing a resulting hydroxy keto ester VIII by methods known per se to the 1,3-diol ester of the formula IX

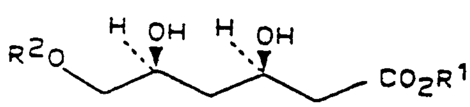

IX

in which $R^1$ and $R^2$ have the meanings indicated for formula VII,

4) converting a resulting 1,3-diol ester of the formula IX by introduction of a protective group suitable for 1,3-diols into a compound of the formula X

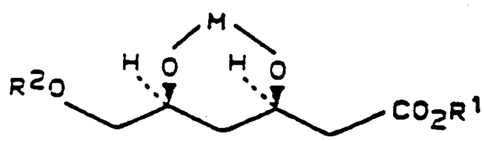

X

in which M is a protective group suitable for 1,3-diols, and $R^1$ and $R^2$ have the meanings indicated for formula VII,

5) converting a resulting compound of the formula X by customary methods with elimination of the protective group $R^2$ into a compound of the formula III

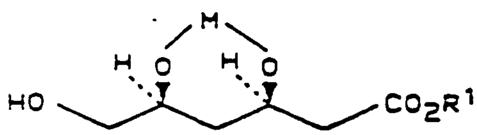

III

in which M is a protective group suitable for 1,3-diols, and $R^1$ denotes a $C_1$-$C_8$-alkyl radical,

6) oxidizing a resulting compound of the formula III to the aldehyde of the formula XII

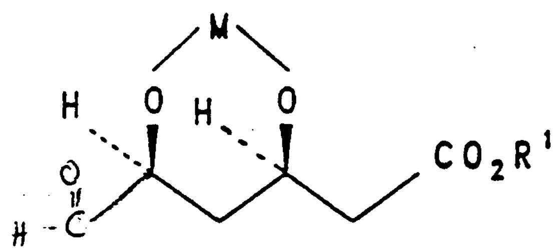

XII

and then converting it into an olefin derivative of the formula XI

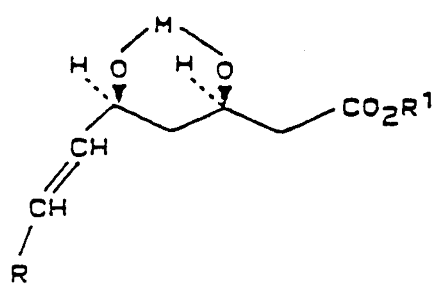

XI

(Y of formula I = CH=CH)

and

7) hydrolyzing a resulting compound of the formula XI to give a compound of the formula I in which Y denotes the CH = CH group, and $R^1$ represents a $C_1$-$C_8$-alkyl radical,

8) where appropriate hydrogenating a resulting compound of the formula I in which Y represents a CH = CH group to give a compound of the formula I in which Y is the $CH_2$-$CH_2$ group,

9) where appropriate converting a resulting compound into the acid ($R^1$ = H) or a salt ($R^1$ = metal cation) and

10) where appropriate converting a resulting compound of the formula I into a compound of the formula II.

**2.** An aldehyde of the formula XII

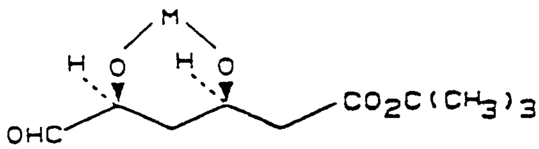

XII

in which M denotes a radical of the formula

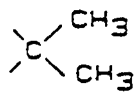 , 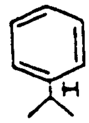 or 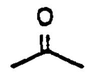

**3.** A process for the preparation of an aldehyde of the formula XII, which comprises oxidation of an alcohol of the formula III in which M has the meanings indicated for formula XII.

**Revendications**

**1.** Procédé pour la préparation de dérivés d'acide 3-desméthylmévalonique optiquement actifs de formule I

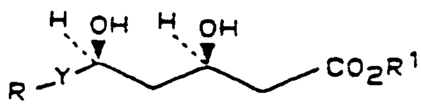

I

(dérivés d'acide 3,5-dihydroxycarboxylique) ou de formule II

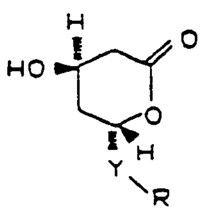

II

($\beta$-hydroxylactones),
formules dans lesquelles
Y est le groupe -CH=CH- ou $-CH_2-CH_2-$,
R représente un radical de formule $\alpha$

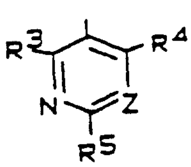

$\alpha$

dans laquelle

| | |
|---|---|
| Z | représente un radical de formule -CH ou un atome d'azote, et |
| $R^3$, $R^4$, $R^5$ | représentent, indépendamment les uns des autres, un atome d'hydrogène, un radical |

hydrocarboné à chaîne droite ou ramifiée ayant jusqu'à 6 atomes de carbone, qui peut éventuellement être substitué, sur l'atome de carbone terminal, par un groupe hydrocarboné cyclique, saturé ou insaturé, ayant de 3 à 6 atomes de carbone, ou représentent un radical hydrocarboné cyclique saturé ou jusqu'à deux fois insaturé, ayant de 3 à 7 atomes de carbone, un radical aromatique choisi parmi les radicaux phényle, furyle, thiényle, pyridinyle, qui peut éventuellement porter sur le noyau 1 à 3 substituants identiques ou différents parmi les substituants suivants: halogène, trifluorométhyle, alkyle ou alcényle ayant chacun jusqu'à 6 atomes de carbone, hydroxy, alcoxy ayant de 1 à 6 atomes de carbone, carboxy, carbalcoxy ayant de 1 à 6 atomes de carbone dans le fragment alcoxy,

ou un radical de formule *β*

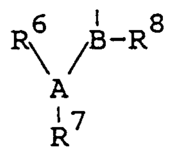

*β*

dans laquelle

A-B représente le groupe -CH-CH- ou -C = C-,

$R^6$ et $R^7$ ($R^6$ et $R^7$ étant identiques ou différents) représentent un radical alkyle saturé ou insaturé ayant jusqu'à 20 atomes de carbone, qui peut être substitué par un groupe alkyle ayant de 1 à 6 atomes de carbone ou par le groupe

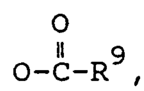

$R^9$ représentant un groupe alkyle ayant de 1 à 8 atomes de carbone,
un radical cycloalkyle ayant de 3 à 7 atomes de carbone, un radical phényle, thiophène, furanne ou naphtalène, les radicaux aromatiques pouvant être substitués 1 à 3 fois sur le noyau par un ou des atomes d'halogène ou groupes alkyle ou alcoxy ayant chacun de 1 à 6 atomes de carbone, cycloalkyle ayant de 3 à 7 atomes de carbone ou par le groupe

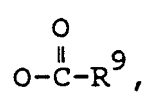

$R^9$ représentant un groupe alkyle ayant de 1 à 8 atomes de carbone,
ou un radical pyridine qui peut être substitué de 1 à 3 fois sur le noyau par un ou des groupes alkyle ayant de 1 à 4 atomes de carbone,

$R^8$ représente un radical alkyle saturé ou insaturé ayant jusqu'à 8 atomes de carbone, un radical benzyle qui peut être substitué de 1 à 3 fois sur le noyau par un ou des atomes d'halogène ou groupes alcoxy ou alkyle ayant chacun de 1 à 4 atomes de carbone, un radical phényle, thiophène, furanne, naphtalène, les radicaux aromatiques pouvant être substitués 1 à 3 fois sur le noyau par un ou des atomes d'halogène ou groupes alcoxy ou alkyle ayant chacun de 1 à 4 atomes de carbone, ou un radical cycloalkyle ayant de 3 à 7 atomes de carbone, et

$R^1$ représente un atome d'hydrogène, un cation métallique ou un groupe alkyle ayant de 1 à 8 atomes de carbone,

caractérisé en ce que

1) on convertit un ester de diol de formule VI

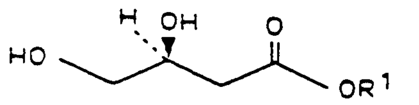

VI

dans laquelle $R^1$ représente un groupe alkyle en $C_1$-$C_8$, par introduction d'un groupe protecteur usuel, selon des méthodes connues en soi, en un composé protégé sur le groupe alcool primaire, de formule VII

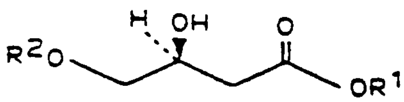

VII

dans laquelle $R^1$ représente un groupe alkyle en $C_1$-$C_8$ et $R^2$ représente un groupe protecteur de fonction alcool usuel,

2) on convertit un composé de formule VII obtenu, ou son alcoolate correspondant, par condensation usuelle avec l'acétate de tert-butyle ou un équivalent approprié, en un composé de formule VIII

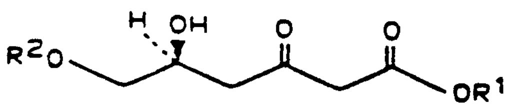

VIII

dans laquelle $R^1$ et $R^2$ ont les significations données à propos de la formule VII,

3) on réduit un hydroxy-cétoester VIII obtenu, selon des méthodes connues en soi, en l'ester de 1,3-diol de formule IX

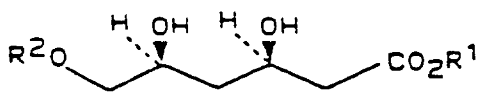

IX

dans laquelle $R^1$ et $R^2$ ont les significations données à propos de la formule VII,

4) on convertit un ester de 1,3-diol de formule IX, par introduction d'un groupe protecteur approprié à des 1,3-diols, en un composé de formule X

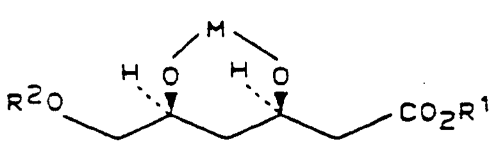

X

dans laquelle M représente un groupe protecteur approprié à des 1,3-diols, et $R^1$ et $R^2$ ont les significations données à propos de la formule VII,

5) on convertit un composé de formule X obtenu, selon des méthodes usuelles, avec élimination du groupe protecteur $R^2$, en un composé de formule III

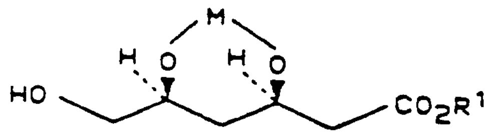

III

dans laquelle M est un groupe protecteur approprié à des 1,3-diols, et $R^1$ représente un radical alkyle en $C_1$-$C_8$,

6) on oxyde un composé de formule III obtenu en l'aldéhyde de formule XII

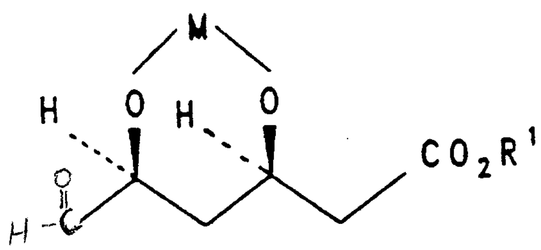

XII

et on le convertit ensuite en un dérivé d'oléfine de formule XI

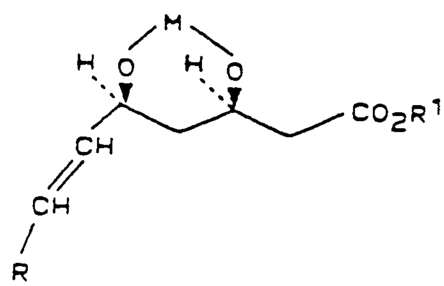

XI

(Y de la formule I = CH=CH) et

7) on hydrolyse un composé de formule XI obtenu en un composé de formule I, dans laquelle Y représente le groupe CH = CH et $R^1$ représente un groupe alkyle en $C_1$-$C_8$,

8) on soumet éventuellement à une hydrogénation un composé de formule I obtenu, dans lequel Y représente un groupe CH = CH, pour aboutir à un composé de formule I dans lequel Y est le groupe $CH_2$-$CH_2$,

9) éventuellement on convertit un composé obtenu en l'acide ($R^1$ = H) ou un sel ($R^1$ = cation métallique) et

10) éventuellement on convertit un composé de formule I obtenu en un composé de formule II.

**2.** Aldéhydes de formule XII

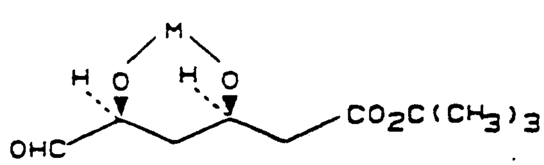

XII

dans laquelle M représente un radical de formule

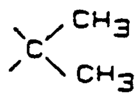, 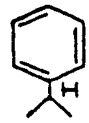 ou 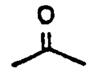

**3.** Procédé pour la préparation d'aldéhydes de formule XII, caractérisé en ce que l'on oxyde des alcools de formule III dans lesquels M a les significations données à propos de la formule XII.